# EUROPEAN PATENT APPLICATION

(11) **EP 4 545 026 A1**
(43) Date of publication of application: **30.04.2025**
(21) Application number: 23306469.0
(22) Date of filing: 05.09.2023
(51) Int. Cl.: A61B 17/435

(54) **OPEN RECOVERABLE INTRA-UTERIN DEVICE**

(71) Applicant: MOCK, Pascal, 1205 Genève (CH)
(72) Inventor: MOCK, Pascal, 1205 Genève (CH)
(74) Representative: Regimbeau

(57) **Abstract**

The present invention relates to an open recoverable intra-uterine device used particularly for culturing gametes and/or embryos ***in utero.***

## Description

### FIELD OF THE INVENTION

The present invention relates to an open recoverable intra-uterine device used particularly for culturing gametes and/or embryos ***in utero.***

### BACKGROUND OF THE INVENTION

In natural conception, floating embryo arrives in the uterine cavity from the Fallopian tube where fertilization happened, at the fourth day of its development (morula) and around 24h before the beginning of implantation. This short period of time before implantation may play a role in the decrease of expulsion risk from the uterine cavity.

In ***in vitro*** fertilization (IVF), however, generally, the embryo is transferred into the uterine cavity at day 2 or 3, i.e., it is present in the uterine cavity 48h to 72h before its presence during the natural conception. Consequently, it remains much more time in a floating state which may contribute to a higher risk to be expelled from the uterine cavity. Different reasons are usually proposed to be implicated in this risk of expulsion including uterine peristalsis and contractions, low site of deposition and negative pressure generated when removing the transfer catheter. Several strategies as bed rest, fibrin sealant and mechanical closure of the cervix were proposed to avoid this problem of embryo expulsion. Unfortunately, all these strategies failed to resolve this problem.

The main reason why the embryo is transferred at day 2 or 3 is the consequence of a persistent technical difficulty to optimize culture medium ***in vitro.*** Indeed, ***in vitro*** culture of mammalian embryos is a fundamental process in assisted reproductive technology (ART). Generally, ***in vitro*** culture conceived for using in ART, only mimics complexity of ***in vivo*** and ***in utero*** microenvironment. However, the natural milieu of gametes and embryos also contains redundant and overlapping reactive oxygen species (ROS)-protective systems that cannot even be mimicked ***in vitro*** (Menezo Y et al. 2016).

Dialogue between mother and embryo through an important intercellular communication network with extracellular vesicles has recently been proposed to play a crucial role in implantation (Godakurama et al. 2022, Javier Gonzalez Fernandez 2023). Whereas extracellular vesicles (EVs) such as apoptotic bodies, microvesicules (MVs), and exosome (EXOs) are released from all cells in the body and are accumulated in all fluid spaces (Beetler et al. 2023), in the reproductive field, they are secreted from the embryo (trophectoderm) and from the maternal endometrium (epithelial cells) measuring between 100 to 1000 nm. They contain lipids, proteins, RNAs (IncRNA, mRNA, small non-coding RNA, rRNA, miRNA) and DNAs. Recently, it was also suggested that endometrium itself may be implicated in the preparation of embryo to implant. First, miR-30d containing EXOs is secreted by endometrium and becomes internalized by embryonic trophoblast cells. It has been shown to induce genes involved in embryonic implantation (Vilella et al., 2015). It was also reported that mtDNAs present in MVs which were secreted by endometrial cells, seems to involved in the embryonic ATP production. All these possible effects from the endometrium on the embryo highlights that the reproductive tract EVs cargo has an important role in reproductive events, which is missing in current ***in vitro*** media.

In addition, the influence of culture conditions on epigenetic chromatine remodelling during pre-implantation development has been demonstrated. Most studies use a Mouse Embryo Assay (MEA) to assess appropriate DNA methylation. One of these studies demonstrated a loss of imprinted methylation among all culture systems using commercial media (used in IVF) compared with ***in vivo***-derived embryos. No commercial human IVF culture media would pass the Mouse Embryo Assay (MEA) if appropriate methylation/epigenetics were considered (Market-Velker BA et al. 2010). In a recent study it was demonstrated that ***in vitro*** culture media are implicated in this alteration of epigenetic remodelling of chromatin in human ART (Yves Ménézo et al., 2020)

In conclusion the exposition of IVF-derived embryos to sub-optimal ***in vitro*** conditions may lead to epigenetic errors with possible adverse short (mosaicism, invasive phenotype) and long term (Developmental Origin of Health and Disease-DOHaD) or even transgenerational consequences (Ventura et al., 2015). In the last 15 years it has been demonstrated in bovine model that the post-fertilization environment affects embryo quality in terms of gene expression, cryotolerance and metabolism (Wrenzycki et al., 2005; Lonergan et al. 2006; Duranthon et al., 2008) with even a change in the DNA methylation profile according to the time of in vitro sub-optimal exposition (Salilew-Wondim et al., 2015).

Considering the difficulty to render the conditions of ***in vitro*** culture closer to the conditions ***in utero,*** notably after gamete intrafallopian transfer (GIFT) and (zygote intrafallopian transfer (ZIFT) with the invasive laparoscopy, the research efforts for improving ***in vitro*** fertilization (IVF) are now directed to selection with artificial intelligence of the best ***in vitro*** derived embryo to be implanted. In addition, preimplantation genetic test to detect aneuploidies (PGT-a) is used as means to improve implantation.

However, all these technics failed to resolve the problem of embryo quality implantation in the uterine cavity. It still remains a need to improve preimplantation embryo developments' conditions to produce embryos more functional with better quality and ability of implantation.

Some studies were directed to the development of means allowing to perform an ***in vivo*/*in utero*** culture. Particularly, three different devices for embryo ***in vivo*** preimplantatory development are known.

One of them (developped by Invocell^{®}) is a vaginal device. It consists in a box as a real container without any link and communication with the environment and containing oocytes and sperm and placed into the vagina. Only temperature, no light and presence of patient's movement are considered to be « in vivo ».

The second one is an intra-uterine device disclosed in the application WO 03/011200**.** It consists in a permeable silicone capsule permitting a communication between its inner and outer part. The post ICSI (intra-cytoplasm sperm injection) oocytes are placed into this capsule and transitory placed into the uterus less than 2 hours later as a mini-intrauterine device (IUD) offering to early preimplantatory embryo a natural environment. The principle of this device is to allows the exchanges between the uterine medium and the embryo/gametes encapsulated in the device via the wall of the device made from a permeable material. However, even if several molecules contained in the uterine medium are able to cross the permeable wall, the exchange of molecules and circulation of the uterine medium is insufficient, and the embryos/gametes placed in the devices are not in optimal culture conditions.

In addition, a permeable porous membrane is fragile and handling of this capsule, in particular when loading and unloading encapsulated elements, or when implanting the capsule in or recovering it from the uterus, could damage the wall of the capsule and possibly cause the loss of the encapsulated elements.

To improve ***in utero*** culture conditions and to optimize the embryos/gametes preimplantation development, a third device was provided (described in the international application WO2007074409), wherein instead to a wall made from permeable material, the device was provided with a wall having several openings with a particular size susceptible to allow molecules of the uterine medium to enter into the device but blocking the exit of embryos/gametes placed in the device. However, even if several molecules of the uterine medium are able to enter the device, there are other one which remain outside and also, other cells necessary to the preimplantation development of the embryo, whose size is bigger than the size of the openings cannot penetrate into the device and the culture conditions are thus not optimal. Finally, the high viscosity of the uterine fluid may explain why exchanges between microenvironment and uterine elements are not optimal with this device.

From the above, it appears that the current methods and devices do not allow to solve the problems occurring during the period of preimplantation of embryo and thus increasing the quality of gametes and embryos and embryo implantation rate after its transfer into uterine cavity. Therefore, there is still a need to provide methods and/or devices allowing to improve the condition of preimplantation in order to optimize the quality of gametes and embryos and to increase the chance of embryo implantation and development in the uterine cavity and thus improving the ART.

### SUMMARY OF THE INVENTION

As it appears from the background part, developing an ***in vitro*** preimplantation embryo culture which mimics closely the ***in vivo*/*in utero*** conditions seems to be a hard work. In addition, the devices already provided for putting into contact embryo/gametes with the uterine medium do not allow to obtain an optimal quality of embryo and consequent rate of implantation. This is the reason why the current studies are rather directed to an improvement of the methods of selecting embryo before the implantation into the uterine cavity instead to the improvement of culture conditions of preimplantation stage, let alone to the improvement of means allowing ***in vivo*/*in utero*** culture.

Surprisingly, in the above-mentioned conditions, the inventor of the present invention decided to continue the effort of improvement of transitory ***in vivo*/ *in utero*** culture for embryo/oocyte development in order to decrease the ***in vitro*** culture time before definitive transfer in the uterine cavity, since this appears to him to be the best route for improving the methods of assisted fertilization and particularly, the functionality of embryo in order to improve the process of implantation. Maintaining the principle of using the uterine cavity as an incubator and the uterine medium as a natural culture medium, the inventor found out a solution allowing to put into very close contact (as during the natural conception), the embryo/gametes with all elements (molecules and cells) of the uterine medium without any restriction. This solution consists in an open recoverable intra-uterine device which is manufactured so as to permit the entry of the molecules and cells contained in the uterine medium into the device but without allowing the embryos/gametes to leave the device and to escape in the uterine cavity.

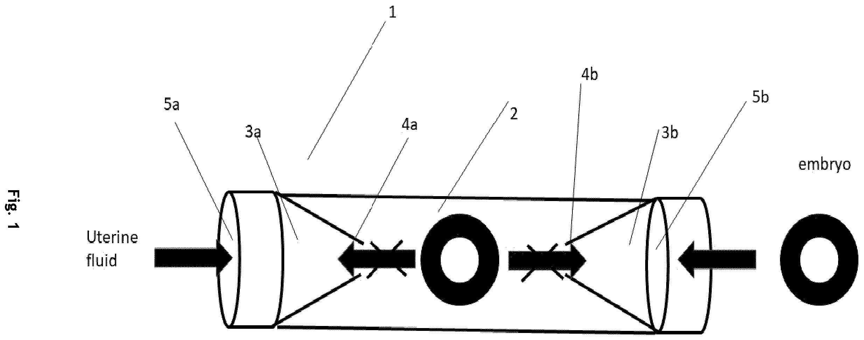

The present invention (Figure 1) thus relates to an open recoverable intra-uterine device (1) adapted to contain one or more elements selected from the group consisting of embryos, male and/or female gametes, fertilized oocytes, unfertilized eggs, or combinations thereof, the housing comprising:
- a housing (2) of biocompatible material, the housing (2) having:two distal openings (3a, 3b) having a conical shape allowing intra-uterine medium to penetrate and circulate into the device and the elements contained in the device to remain retained therein.

Thus, according to the invention, the device comprises an opening allowing the uterine fluid even with its high viscosity with all its components (molecules and cells) to penetrate into the housing and to circulate within, being thus in close cellular contact with the elements contained in the device.

In addition, the specific conical shape of the opening allows uterine fluid with all its components to enter into the housing of the device but do not allow the elements loaded in the housing to leave into the uterine cavity.

The entry into the housing of the device of the uterine fluid, which may comprise fluids coming from both, the uterine cavity and from the fallopian tubes, can favor the development of the embryos. Furthermore, exchange of cells can facilitate subsequent re-implantation and nidation of the embryo in the uterus.

The phenomena of interaction with the uterine fluid and all its components, enhanced compared to the state of the art, can be of a kind to favor the development of the elements contained in the housing, and in particular the development of the embryo or the fertilization of the implanted eggs.

In addition, the open recoverable intra-uterine device of the invention is able i) to improve embryo quality and invasive phenotype and euploidy level; ii) to respect this high vulnerability period of embryo development with genome reprogramming; iii) to increase involvement of future parents, mother's uterine cavity being used as natural biological incubator; iv) to ensure close contact with cells and uterine fluid offering real cross-talk between the mother and the embryo; v) to offer a period of rescue and wellness to embryos before or after all sophisticated handlings such as ICSI, vitrification or ***in vitro*** culture.

### DETAILED DESCRIPTION OF THE INVENTION

Other features and advantages of the open intra-uterine device of the invention will become more apparent in the course of the following description.

In the appended drawing, provided by way of non-limiting example, FIG. 1 shows a diagrammatic perspective view of open recoverable intra-uterine device (1) of the invention.

The open recoverable intra-uterine device shown in **FIG.1** is particularly used in assisted fertilization techniques to implant and temporarily maintain male and female gametes **(*in vivo*** fertilization) and/ or embryos (preimplantation development) in a uterine cavity.

In principle, in an ***in vitro*** assisted fertilization method, this intra-uterine device enables one or more elements to be loaded into a housing (2) at the start of development, for example the embryo in the start of embryo development. The device is introduced into the uterine cavity for a predetermined period (from a few hours to a few days), and thereafter it is recovered to extract the embryo(s) in order to implant them in the uterine cavity or fertilized oocytes in diagnosis purpose before intra-uterine implantation.

The open recoverable intra-uterine device (1) of the invention comprises a housing (2) of biocompatible material and comprising two openings (3a, 3b), particularly two lateral openings, placed one on the left (3a) and one the right (3b) extremity of the housing. These openings have a conical shape allowing the loading of elements (embryos, male and/or female gametes, fertilized oocytes, unfertilized eggs) into the housing and the entry of the uterine fluid with its components.

The open recoverable intra-uterine device may take any shape which will accommodate gametes and/or embryos or any other above-mentioned elements to be loaded in the device.

According to one embodiment shown in **FIG.1** the housing (2) has a cylindrical shape and is preferably a tube. In this embodiment, the outer diameter of this housing is between 700 µm and 1 cm, to be loaded easily into a standard transfer catheter and go through the cervix. The inner diameter of the housing is between 300 µm and 600 µm adapted to the size of the mammalian, preferably human egg which measures, including the corona radiata about 200 µm, and is preferably equal to 600 µm. The length of the housing is between 1 cm and 1,5 cm, length adapted to the uterine cavity avoiding endometrial lesions and any deleterious effects caused by uterine distension, such an expulsion of the device.

As indicated above, the open recoverable intra-uterine device of the invention comprises two opening (3a, 3b) having a conical shape with narrow part (4a, 4b) and with wide part (5a, 5b) allowing intra-uterine medium to penetrate into the device and the elements contained in the housing to remain retained in the housing.

The function of the conical shape of the opening follows the principle of a trap, i.e. it allows uterine fluid to penetrate and to circulate in the housing and the elements to be loaded in the housing but does not allow these elements to leave the housing.

Furthermore, the size of diameter of the narrow part (4a, 4b) and the size of diameter of the wide part (5a, 5b) of the opening (3a, 3b) is selected so as to allow that the uterine fluid with all of its components penetrates in the housing and that the elements loaded within the housing to be retained.

The conical opening has a wide part (5a, 5b) with a diameter comprised between 300 µm and 600 µm corresponding to the inner diameter of the housing element, a narrow part (4a, 4b) with a diameter comprised between 50 and 150 µm avoiding any expulsion of loaded elements (especially eggs with a size of 200 micrometers) outside the housing and a length comprised between 300 µm and 500 µm.

Particularly, the diameter of the narrow part (4a, 4b) of the opening is comprised between 80 µm and 140 µm and preferably, between 100 µm and 130 µm.

According to the embodiment shown on **FIG. 1****,** the housing (2) comprises two lateral openings (3a, 3b) whose narrow part (4a, 4b) is oriented towards the inside of the device and the wide part (5a, 5b) towards the outside of the device. Particularly the housing is a tube, and the openings have sufficient size to allow a free circulation of intra-uterine medium through the device and to retain the loaded elements

The embodiment is particularly advantageous since it allows the circulation of the uterine fluid and its components inside the housing and also, a discontinuous exchange between the inner part of the housing and the loaded elements and the surrounding uterine fluid.

According to one embodiment, the uterine fluid penetrates in the housing of the device from at least one of both openings (3a, 3b) and circulates toward the opposite opening. Preferably, the uterine fluid penetrates from the two openings and circulates toward the opposite opening, i.e., from left opening to right opening and/or from right opening to left opening.

The housing of the open recoverable intra-uterine device can be constituted from any one of materials selected from the group consisting of a polymer, a ceramic, a glass, an elastomeric material, a metal material, a stainless steel, a titanium and a titanium alloy, preferably a titanium or a titanium alloy.

In particular, this housing can be constituted from a porous polymer, polyethersulfone (PES), polyacrylate, acry-late copolymer or polyvinylidiene type.

Generally speaking, the materials used must be tested to be non-toxic, biocompatible and stable in use.

According to one embodiment, the conical shape opening may be produced in material different from those used for producing the wall of the housing.

According to one preferred embodiment, the material used for producing the openings is a shape memory material. In the context of the present invention, **"a shape memory material"** refers to a material which can be deformed, generally when cold but returns to its pre-deformed ("remembered") shape, generally when heated. The shape memory material is selected from shape memory alloy, shape memory polymers or shape memory composites.

The conical shape of the opening of the housing of the device of the invention as defined above is sufficient to ensure that the elements loaded in the housing will remain inside after introducing the device in the uterine cavity.

As indicated above, the elements loaded and/or attached in the housing of the device of the invention are selected from the group consisting of embryos, male and/or female gametes, fertilized oocytes, unfertilized eggs, or combinations thereof, particularly embryos. Particularly, these elements are obtained from mammal, particularly vomprising bovine, ovine, porcine, horses and human and more particularly, from a human being. In accordance with a preferred embodiment, the loaded element is a human embryo.

These elements are loaded in the housing via conventional means such as standard micropipette for loading embryos.

The device of the present invention is capable of being placed within the uterine cavity of a mammal, selected from the group consisting of bovine, ovine, porcine, horses and human, preferably human.

The present invention also relates to a method of preparing an open retrievable intra-uterine device for placing one or more elements selected from the group consisting of embryos, male and/or female gametes, fertilized oocytes, unfertilized eggs, or combinations thereof, preferably an embryo and more preferably a human embryo comprising the steps of:
- providing said element under the appropriate form to be introduced in the device,
- providing the open retrievable intra-uterine device suitable for receiving said element(s) according to the invention,
- loading said device with said element(s).

The open recoverable intra-uterine device of the invention furthermore cooperates with accessories for placing the device in the uterine cavity. For example, a transfer catheter having an inside diameter of 1.1 cm to 1.3 cm may be used. A catheter of this kind is adapted to pass through the cervical canal, which generally has a diameter of about 1.5 cm. The oocytes, surrounded by complex cells, developing in the housing have a size between 350 and 400 µm, and so a housing having an inner diameter of 600 µm is sufficient to contain between 5 and 10 oocytes.

As indicated above, the device of the invention can be implanted passing through the cervix as a standard IUD (intra-uterine device) for contraception into the uterus and removed after a defined time of incubation.

The device of the present invention may also use as implantation site the Fallopian tube. This implantation needs to use a surgical procedure as coelioscopy with general anesthesia or culdoscopy with local anaesthesia. Such ***in vivo*** intra Fallopian embryo culture is similar to GIFT or ZIFT except the fact that using of the open recoverable intra-uterine device of the invention, the incubation time is under control and an unlimited number of embryos can be loaded which were retrieved and selected for transfer after a simple flushing procedure.

The device of the invention presents a number of advantages: said device is not likely to cause any trouble to the uterus (little or no tissue reaction, without inducing inflammatory or fibrotic reactions and or inappropriate uterine wall tissue damage or scarring) due to the fact that it is not implanted within the uterine wall, it does not require absolutely surgery or anaesthesia to be inserted and it can be inserted in a completely ambulatory fashion.

Furthermore, the device of the invention is designed such that it has a means of remaining within the uterus (i.e. small suture thread glued into the tip of the device for attachment inside or outside the uterus) and that can be easily retrieved (attached suture thread) at any time after uterine implantation.

As explained above, the open recoverable intra-uterine device of the invention is able to present gametes and/or embryos to the natural microenvironment of the uterine cavity without encapsulation (as the similar devices in the relating art). It permits to obtain for the gametes and the embryos an optimal micro-environment in a small volume. The device of the invention offers to developing embryos a closer biophysics-chemical environment with less osmolarity alteration and permits to the uterus to become a real transitory natural incubator offering the possibility of a complete physico-chemical and cellular complex cross talk to take place between the mother and embryos. Even, complete ***in vivo*/*in utero*** fertilization may take place into the uterine cavity with the performance of intrauterine insemination after the intrauterine transfer of this device fixing oocytes.

The device of the present invention resolves the problem of embryo(s) passage from the uterus through the inner cervical canal and their expulsion into the vagina.

Therefore, as mentioned above, the open recoverable intra-uterine device may be used successfully in assisted reproduction.

According to one aspect, the present invention thus relates to a method of assisted reproduction, wherein the method comprises the following steps:
- preparing an open recoverable intra-uterine device, particularly as per the above-described method,
- placing the device in the uterine cavity for a predetermined period,
- at the end of this period, removing the device,
- monitoring the development of the element of interest, and
- reintroducing the element of interest into the uterine cavity without the device of the invention or conserving the element for further treatment.

As used herein, the **"predetermined period"** corresponds to the period or the time necessary to postfertilization embryo development (conventionally performed in ***in vitro*** culture) before the definitive transfer of the embryo into uterine cavity. The "predetermined period" may also correspond to the time necessary for performing ***in utero*** fertilization (i.e. fertilization in the device of the invention) when oocytes and spermatozoids are contained in the device.

The device may be placed in the uterine cavity by the means described above.

More particularly, the device of the present invention may be used in ***in vivo*** fertilization by injecting prepared sperm and retrieval oocytes in the device for implanting it into the uterus. After a defined and controlled incubation time (2 hours for example) of ***in vivo*** and ***in utero*** culture, the sperm and oocytes are retrieved and zygotes and/or unfertilized oocytes are collected after a simple flushing procedure. Then, selection of zygotes for cryopreservation or ***in vitro*** culture of the remaining embryos to be transferred at day 3 is performed.

According to one embodiment, the device of the present invention may be also used in ***in vivo*** preimplantatory embryo development by injecting several embryos at different stage of development (i.e 6-8 cells) in the device followed by the implantation of the device into the uterine cavity during a controlled time (i.e 48 hours). After removal of the device from the uterus, the embryos at the blastocyst stage are flushed from the device and transferred into the uterine cavity using a conventional transfer catheter or delayed in a further cycle after freezing.

According to another embodiment, the device of the invention may be used in ***in vivo*** embryo assisted hatching.

Of course, numerous modifications can be made to the embodiment described hereinabove without departing from the scope of the invention.

### BIBLIOGRAPHICAL REFERENCES

Beetler DJ, Di Florio DN, Bruno KA, Ikezu T, March KL, Cooper LT Jr, Wolfram J, Fairweather D. Extracellular vesicles as personalized medicine. Mol Aspects Med. 2023 Jun;91:101155. doi: 10.1016/j.mam.2022.101155. Epub 2022 Nov 28. PMID: 36456416; PMCID: PMC10073244.
Duranthon V, Watson AJ, Lonergan P. Preimplantation embryo programming: transcription, epigenetics, and culture environment. Reproduction. 2008 Feb;135(2):141-50. doi: 10.1530/REP-07-0324. PMID: 18239045.
Godakumara K, Dissanayake K, Hasan MM, Kodithuwakku SP, Fazeli A. Role of extracellular vesicles in intercellular communication during reproduction. Reprod Domest Anim. 2022 Oct;57 Suppl 5(Suppl 5):14-21. doi: 10.1111/rda.14205. Epub 2022 Jul 24. PMID: 35837748; PMCID: PMC9 796405.
Gonzalez Fernandez J, Moncayo Arlandi J, Ochando A, Simon C, Vilella F. The role of extracellular vesicles in intercellular communication in human reproduction. Clin Sci (Lond). 2023 Feb 14;137(3):281-301. doi: 10.1042/CS20220793. PMID: 36762584.
Lonergan P, Fair T, Corcoran D, Evans AC. Effect of culture environment on gene expression and developmental characteristics in IVF-derived embryos. Theriogenology. 2006 Jan 7;65(1):137-52. doi: 10.1016/j.theriogenology.2005.09.028. Epub 2005 Nov 9. PMID: 16289260.
Market-Velker BA, Fernandes AD, Mann MR. Side-by-side comparison of five commercial media systems in a mouse model: suboptimal in vitro culture interferes with imprint maintenance. Biol Reprod. 2010 Dec;83(6):938-50. doi: 10.1095/biolreprod.110.085480. Epub 2010 Aug 11. PMID: 20702853.
Ménézo Y, Elder K. Epigenetic remodeling of chromatin in human ART: addressing deficiencies in culture media. J Assist Reprod Genet. 2020 Aug;37(8):1781-1788. doi: 10.1007/s10815-020-01884-6. Epub 2020 Jul 16. PMID: 32676929; PMCID: PMC7468041.
Min JY, Jang YJ. Use of 2-octylcyanoacrylate (Dermabond) tissue adhesive for tip graft fixation in open rhinoplasty. Otolaryngol Head Neck Surg 2011;145:737-41
Oriol Penon et al. Efficient biofunctionalization of polysilicon barcodes for adhesion to the zona pellucida of mouse embryos. Bioconjugate Chem. 2012, 23, 2392-2402.
Salilew-Wondim D, Fournier E, Hoelker M, Saeed-Zidane M, Tholen E, Looft C, Neuhoff C, Besenfelder U, Havlicek V, Rings F, Gagné D, Sirard MA, Robert C, Shojaei Saadi HA, Gad A, Schellander K, Tesfaye D. Genome-Wide DNA Methylation Patterns of Bovine Blastocysts Developed In Vivo from Embryos Completed Different Stages of Development In Vitro. PLoS One. 2015 Nov 4;10(11):e0140467. doi: 10.1371/journal.pone.0140467. PMID: 26536655; PMCID: PMC4633222.
Ventura-Juncá P, Irarrázaval I, Rolle AJ, Gutiérrez JI, Moreno RD, Santos MJ. In vitro fertilization (IVF) in mammals: epigenetic and developmental alterations. Scientific and bioethical implications for IVF in humans. Biol Res. 2015 Dec 18;48:68. doi: 10.1186/s40659-015-0059-y. PMID: 26683055; PMCID: PMC4684609.
Vilella F, Moreno-Moya JM, Balaguer N, Grasso A, Herrero M, Martinez S, Marcilla A, Simón C. Hsa-miR-30d, secreted by the human endometrium, is taken up by the pre-implantation embryo and might modify its transcriptome. Development. 2015 Sep 15;142(18):3210-21. doi: 10.1242/dev.124289. PMID: 26395145.
Wrenzycki C, Herrmann D, Lucas-Hahn A, Gebert C, Korsawe K, Lemme E, Carnwath JW, Niemann H. Epigenetic reprogramming throughout preimplantation development and consequences for assisted reproductive technologies. Birth Defects Res C Embryo Today. 2005 Mar;75(1):1-9. doi: 10.1002/bdrc.20035. PMID: 15838918.

## Claims

1. An open recoverable intra-uterine device (1) adapted to contain one or more elements selected from the group consisting of embryos, male and/or female gametes, fertilized oocytes, unfertilized eggs, or combinations thereof, the device comprising:
- a housing (2) of biocompatible material, the housing (1) having two distal openings (3a, 3b) having a conical shape allowing intra-uterine medium to penetrate and circulate into the device and the elements contained in the device (1) to remain retained therein.

2. The open recoverable intra-uterine device (1) according to claim 1, wherein the housing (2) has a cylindrical shape.

3. The open recoverable intra-uterine device (1) according to claim 2, wherein the housing (2) is a tube.

4. The open recoverable intra-uterine device (1) according to any one of claims 1 to 3, wherein the outer diameter of the housing (2) is between 700 µm and 1 cm, the inner diameter of the housing is between 300 µm and 600 µm and the length of the housing is between 1 cm and 1,5 cm.

5. The open recoverable intra-uterine device (1) according to any one of claim 1 to 4, wherein the conical opening has a narrow part (4a, 4b) with a diameter comprised between 50 µm and 150 µm, a wide part (5a, 5b) with a diameter comprised between 300 µm and 600, and a length of the opening is comprised between 300 µm and 500 µm.

6. The open recoverable intra-uterine device (1) according to claim 5, wherein the diameter of the narrow part (4a, 4b) of the opening is comprised between 80 µm and 140 µm and preferably between 100 µm and 130 µm.

7. The open recoverable intra-uterine device (1) according to any one of claims 1 to 6, wherein the narrow part (4) of the opening (3a, 3b) is oriented towards the inside of the device and the wide part (5a, 5b) of the opening is oriented towards the outside of the device.

8. The open recoverable intra-uterine device (1) according to any one of claims 1 to 7, wherein the openings are manufactured of shape memory material.

9. The open recoverable intra-uterine device (1) according to any one of claims 1 to 8, wherein the biocompatible material is selected from the group consisting of a polymer, a ceramic, a glass, an elastomeric material, a stainless steel, a metal material, a titanium and a titanium alloy, preferably a titanium or a titanium alloy.

10. The open recoverable intra-uterine device (1) according to claim 9, wherein the polymer is selected from the group consisting of porous polymer, polyethersulfone (PES), polyacrylate, acry-late copolymer or polyvinylidiene.

11. The open recoverable intra-uterine device (1) according to anyone of claims 1 to 10, wherein said device is loaded with an embryo.

12. The open recoverable intra-uterine device (1) according to anyone of claims 1 to 11, wherein said device is capable of being placed within the uterine cavity of a mammal, selected from the group consisting of bovine, ovine, porcine, horses and human.

13. The open recoverable intra-uterine device (1) according to claim 12, wherein the mammal is a human.

14. A method of preparing an open retrievable intra-uterine device for placing one or more elements selected from the group consisting of embryos, male and/or female gametes, fertilized oocytes, unfertilized eggs, or combinations thereof comprising the steps of:
- providing said element under the appropriate form to be introduced in the device,
- providing the open retrievable intra-uterine device (1) according to any one of the claims 1 to 13, and
- loading said device with said element(s).

15. The method according to claim 14, wherein the loaded element is an embryo, preferably a human embryo.
